Europäisches Patentamt

European Patent Office

Office européen des brevets

(19)

(11) Publication number: **0 193 973**
**B1**

# EUROPEAN PATENT SPECIFICATION

(12)

(45) Date of publication of patent specification :
06.07.88

(21) Application number : 86104200.0

(22) Date of filing : 04.03.83

(60) Publication number of the earlier application in accordance with Art. 76 EPC : 0117882

(51) Int. Cl.⁴ : **C 07 D239/26**, C 07 C103/44, C 07 D239/06

(54) Method of preparation of 2-alkylpyrimidine by dehydrogenation of 2-alkyltetrahydropyrimidine.

(43) Date of publication of application :
10.09.86 Bulletin 86/37

(45) Publication of the grant of the patent :
06.07.88 Bulletin 88/27

(84) Designated contracting states :
BE CH DE FR GB IT LI NL

(56) References cited :
US-A- 4 493 929
CHEMICAL ABSTRACTS, vol. 85, 1976, pages 462-463, no. 142251m, Columbus, Ohio, US; J. OKADA et al.: "Formation of 2-ethylpyrimidine from trimethylenediamine over platinum group metal-aluminum oxide catalysts and its kinetics study"
CHEMICAL ABSTRACTS, vol. 87, 1977, page 515, no. 84049e, Columbus, Ohio, US; M. TSUCHIYA et al.: "Syntheses of 2-alkylpyrimidines by dehydrocyclization and dehydrogenation"
The file contains technical information submitted after the application was filed and not included in this specification

(73) Proprietor : THE DOW CHEMICAL COMPANY
2030 Dow Center Abbott Road P.O. Box 1967
Midland, MI 48640 (US)

(72) Inventor : Pews, Richard Garth
4403 Andre
Midland Michigan 48640 (US)

(74) Representative : Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel
Postfach 860820
D-8000 München 86 (DE)

## Description

The preparation of tetrahydropyrimidines is discussed in « The Pyrimidines », Interscience (1962), page 445 ff. The preferred reaction involved refluxing 1,3-diaminopropane or a derivative with an alkyl ester of an organic acid, e.g., ethyl benzoate. However, when methyl pivalate was tried in this reaction, hydrolysis occurred and the resulting product was a complex mixture.

The tetrahydropyrimidines and particularly 2-t-butyltetrahydropyrimidine, are advantageously employed as precursors in the preparation of O-alkyl-O-[pyrimidin(5)yl]-(thiono) (thiol)-phosphoric(phosphonic)-acid esters or ester amides having exceptional insecticidal activity by the processes disclosed in, for example, U.S. Patent 4 127 652.

It has now been found that 3-aminopropyl-pivalamide can be prepared in high yields by contacting pivalic acid with 1,3-diaminopropane at temperatures above the boiling point of the 1,3-diaminopropane. Removal of the unreacted 1,3-diaminopropane by distillation and dilution of the reaction mixture with a suitable solvent followed by refluxing provides 2-t-butyl-1,4,5,6-tetrahydropyrimidine in high yields.

The 3-aminopropylpivalamide prepared in this reaction is a novel compound as is N,N'-1,3-propandiylbis (2,2-dimethylpropanamide) which is formed as a by-product. The 3-aminopropylpivalamide is advantageously employed to make 2-t-butyltetrahydropyrimidine which is an intermediate in the preparation of compounds having exceptional insecticidal activity.

The 3-aminopropylpivalamide is prepared by reacting pivalic acid with an excess of 1,3-diaminopropane at a temperature of 110° to 240 °C, preferably at 200° to 225 °C, for 3 to 12 jours. After removal of the unreacted 1,3-diaminopropane by distillation, the residue may be diluted with a suitable solvent and the resulting mixture refluxed, with or without pressure, at temperatures of 110° to 240 °C, peferably 170° to 190 °C, to provide 2-t-butyl-1,4,5,6-tetrahydropyrimidine.

Suitable solvents include those inert solvents which form azeotropes, thereby being useful to remove water from the reacting system, and have a boiling point of 110° to 240 °C at atmospheric conditions, or other inert solvents which will boil at 110° to 240 °C under suitable pressure. Advantageously, the solvent is toluene, o-xylene or diethylbenzene, although chlorinated benzenes such as monochlorobenzene and o-dichlorobenzene may be employed if desired.

A 3 to 10 mole excess of 1,3-diaminopropane is usually employed, although larger molar excesses may be used, if desired. If less than a 3 molar excess is utilized, yields tend to be somewhat lower.

It has further been found that 2-t-butyl-pyrimidine, and other 2-alkylpyrimidines, can be prepared in high yields and purity in a simple one step reaction which comprises the dehydrogenation of a 2-alkyltetrahydropyrimidine under conditions which do not generate water and in which no water is added over a supported noble metal catalyst.

This reaction may be carried out neat, i.e., without employing a solvent, although an inert solvent such as, for example, pyridine may be advantageously employed.

The preferred catalysts are platinum and palladium which are supported on, for example, silica gel, charcoal, activated carbon, magnesia or, preferably, alumina.

The reaction temperature is in the range of 250° to 450 °C, preferably 300° to 400 °C. Advantageously, the desired tetrahydropyrimidine, with or without solvent, is passed through a packed column containing the supported noble metal catalyst heated to the desired temperature. Feed rates to the vapor phase reactor, e.g., a 1″ × 20″ (2.5 × 51 cm) column containing 50 g of catalyst, are 20 to 150 ml/hr, preferably 40 to 80 ml/hr.

To avoid the generation of water in the presence of hydrogen it is necessary that oxygen be excluded from the reaction zone. The exclusion of oxygen is preferably accomplished by sweeping out the reactor with an inert gas, e.g. nitrogen or carbon dioxide. If desired, the inert gas may contain hydrogen gas which serves to activate the catalyst. In any event, hydrogen will be present as the dehydrogenation reaction proceeds.

The invention is further illustrated by the following examples.

### Example 1

A mixture of pivalic acid (136 g, 1.33 mol) and 1,3-diaminopropane (880 ml, 7.75 mol) was heated overnight at 225 °C in a stirred Parr reactor. After cooling and removal of the unreacted 1,3-diaminopropane by distillation, gas chromatographic analysis of the residue, a viscous oily material which was not stable to purification by distillation, showed a 90 % yield of 3-aminopropylpivalamide.

The residue was diluted with o-xylene (250 ml) and the mixture refluxed for 48 hours in a Dean and Stark apparatus to effect cyclization-dehydration. After separation of the azeotrope, the o-xylene was evaporated to give 167 g (90 % yield) of 2-t-butyl-tetrahydropyrimidine having a melting point of 133° to 135 °C.

### Example 2

A mixture of 25.5 g (0.25 mole) of trimethylacetic acid and 100 ml (112.6 g = 1.52 moles) of 1,3-diaminopropane was heated for 16 hours at 200°-210 °C. After cooling and removing the excess 1,3-diaminopropane, the residue, 3-aminopropylpivalamide, a viscous oily material, was analyzed by NMR and gave a spectrum consistent with that expected for 3-aminopropylpivalamide.

A portion (1/2) of the above residue was allowed to react with an equivalent of each of triethylamine and trimethylacetyl chloride at ambient temperature, the product was isolated by extraction with methylene chloride, washed three times with water, dried over $MgSO_4$, the solvent evaporated and the product crystallized on cooling. The filtered and dried product, N,N'-1,3-propandiylbis (2,2-dimethyl-propanamide), 18.5 g, had a melting point of 120°-122 °C, 93.6 %. The NMR spectrum was identical to that of a sample prepared directly from 1,3-diaminopropane and trimethylacetyl chloride.

Analysis :
Calculated for $C_{13}H_{26}N_2O_2$ : C 64.46 H 10.74 N 11.57
Found : C 64.50 H 11.02 N 11.60

## Example 3

A solution of 70 g of 2-isopropyl-1,4,5,6-tetrahydropyrimidine in 100 ml of pyridine was fed at about 1 ml/min to a column 1″ × 20″ containing about 50 g of 0.5 percent platinum on a-alumina at 300° to 325 °C while sweeping out the column with a mixture of nitrogen and hydrogen. The catalyst had been activated by passing a 2 : 1 $H_2/N_2$ stream over the bed for two hours. The effluent was distilled giving 2-isopropylpyrimidine at 152° to 155 °C. The yield was 73 percent.

The above example was repeated employing varying amounts of 2-tertiarybutyl-1,4,5,6-tetrahydropyrimidine, varying amounts of solvents, 0.5 percent palladium on alumina and temperatures of from 250° to 420 °C. The yields of 2-tertiarybutylpyrimidine ranged from 58 to 86 percent.

Similar results are obtained employing other solvents such as, for example, quinoline.

## Example 4

91.6 g of 2-isopropyl-1,4,5,6-tetrahydropyrimidine was fed dropwise to the vapor phase dehydrogenator (1″ × 20″) with 50 g of 0.5 percent palladium on a-alumina over a two hour period while sweeping out he dehydrogenator with a stream of nitrogen and hydrogen. The reactor temperature was 310 °C. The product was distilled to give 67 g of product, b.p. 155 °C, yield 86 percent.

## Claims

1. A process for making 2-alkylpyrimidines which comprises dehydrogenating a 2-alkyltetrahydropyrimidine in a vapor phase reactor in the absence of oxygen and in which no water is added over a supported noble metal catalyst.

2. Process of Claim 1, wherein the 2-alkyltetrahydropyrimidine is 2-isopropyl- or 2-tertiarybutyltetrahydropyrimidine.

3. Process of Claim 2 wherein the reaction is carried out at 250° to 450 °C.

4. Process of Claims 1-3 wherein the noble metal catalyst is supported on charcoal.

5. Process of Claim 4 wherein the noble metal catalyst is palladium.

6. Process of Claim 4 wherein the noble metal catalyst is platinum.

7. Process of Claim 3 wherein the catalyst is platinum or palladium supported on alumina.

## Patentansprüche

1. Verfahren zur Herstellung von 2-Alkylpyrimidinen, umfassend Dehydrieren eines 2-Alkyltetrahydropyrimidins in einem Dampfphasen-Reaktor, in Abwesenheit von Sauerstoff und wobei kein Wasser zugegeben wird, über einem auf einem Träger vorliegenden Edelmetall-Katalysator.

2. Verfahren nach Anspruch 1, worin das 2-Alkyltetrahydropyrimidin 2-Isopropyl- oder 2-Tertiärbutyltetrahydropyrimidin ist.

3. Verfahren nach Anspruch 2, worin die Reaktion bei 250° bis 450 °C ausgeführt wird.

4. Verfahren nach den Ansprüchen 1 bis 3, worin der Edelmetall-Katalysator auf Kohle als Träger vorliegt.

5. Verfahren nach Anspruch 4, worin der Edelmetall-Katalysator Palladium ist.

6. Verfahren nach Anspruch 4, worin der Edelmetall-Katalysator Platin ist.

7. Verfahren nach Anspruch 3, worin der Katalysator Platin oder Palladium auf Aluminiumoxid als Träger ist.

## Revendications

1. Procédé de préparation de 2-alkylpyrimidines, qui comprend la déshydrogénation d'une 2-alkyltétrahydropyrimidine dans un réacteur en phase vapeur, en l'absence d'oxygène, et dans lequel on n'ajoute pas d'eau, sur un catalyseur à métal noble sur support.

2. Procédé selon la revendication 1, dans lequel la 2-alkyltétrahydropyrimidine est la 2-isopropyl- ou la 2-tertiobutyltétrahydropyrimidine.

3. Procédé selon la revendication 2, dans lequel la réaction est réalisée à 250°-450 °C.

4. Procédé selon les revendications 1 à 3, dans lequel le catalyseur à métal noble est supporté sur du charbon.

5. Procédé selon la revendication 4, dans lequel le catalyseur à métal noble est du palladium.

6. Procédé selon la revendication 4, dans lequel le catalyseur à métal noble est du platine.

7. Procédé selon la revendication 3, dans lequel le catalyseur est du platine ou du palladium supporté sur alumine.